# EUROPEAN PATENT SPECIFICATION

(11) **EP 4 355 190 B1**
(45) Date of publication and mention of the grant of the patent: **19.03.2025**
(21) Application number: 22729740.5
(22) Date of filing: 15.06.2022
(51) Int. Cl.: A61B 1/00, A61B 1/07, A61B 1/06, G02B 23/24

(54) **AN ENDOSCOPE**
ENDOSKOP
ENDOSCOPE

(30) Priority: 15.06.2021 EP 21179543
(43) Date of publication of application: 24.04.2024
(73) Proprietor: Ambu A/S, 2750 Ballerup (DK)
(72) Inventor: JOHNSEN, Lasse Markworth, 2750 Ballerup (DK); SØRENSEN, Morten, 2750 Ballerup (DK); LUNDBECH, Michael René, 2750 Ballerup (DK)
(74) Representative: AWA Denmark A/S
(86) International application number: PCT/EP2022/066297
(87) International publication number: WO 2022/263506

(56) References cited:
- EP-A1- 3 539 445
- EP-A1- 3 613 326
- EP-A1- 3 788 940
- US-A1- 2005 075 538

## Description

This application claims priority from and the benefit of European Patent Application No. EP21179543, filed June 15, 2021.

### TECHNICAL FIELD

The disclosure relates to insertable medical vision devices, in particular disposable insertion endoscopes, and more specifically to a housing for the tip of the disposable insertion endoscope and the manufacture thereof.

### BACKGROUND

Vision devices, such as insertion endoscopes, are well known devices for visually inspecting body cavities, such as human body cavities. Typically, an insertion endoscope comprises an elongated insertion tube with a handle at the proximal end as seen from the operator and visual inspections means, such as a built-in camera, at the distal end of the elongated insertion tube. Electrical wiring for the camera and other electronics such as LED lighting accommodated in the tip part at the distal end run along the inside of the elongated insertion tube from the handle to the tip part. Instead of using cameras, endoscopes may also be fibre-optic, in which case the optical fibres run along inside of the elongated insertion tube to the tip part.

In order to be able to manoeuvre the endoscope inside the body cavity, the distal end of the endoscope may comprise a bending section with increased flexibility, e.g. a number of articulated segments of which the tip part forms the distalmost segment. This is typically done by tensioning or slacking pull wires also running along the inside of the elongated insertion tube from the tip part through the remainder of articulated segments to a control mechanism of the handle. Furthermore, a working channel may run along the inside of the insertion tube from the handle to the tip part, e.g. allowing liquid to be removed from the body cavity or allowing the insertion of surgical instruments or the like into the body cavity.

As the name indicates, endoscopes, are used for seeing inside things, such as lungs or other human body cavities of a patient. Modern endoscopes are therefore typically equipped with a least one camera or similar image capturing device at the distal tip of the endoscope. Provided that sufficient light is present, this allows the operator to see where the endoscope is steered and to set the target of interest once the tip has been advanced thereto. This therefore normally requires illumination of the area in front of the distal tip of the endoscope, in particular the field of vision of the camera(s). One known way of achieving such illumination is to provide the above mentioned LED lighting using one or more Light Emitting Diodes (LEDs) in the tip of the endoscope, as e.g. mentioned in commonly-owned U.S. Patent No. 10,321,804, disclosing a disposable endoscope.

When, as according to the present disclosure, the insertion tube of the endoscope is intended to be inserted into a human body cavity, the insertion tube needs to be sealed in a watertight manner. This is in particular the case for the distal tip part because it accommodates the camera, LED(s) and other delicate electronics, prone to malfunction or destruction if exposed to humidity. Also, there are electrical requirements to ensure that the electrical insulation of the endoscope is not compromised e.g. by electrical breakdown of the polymer material from which disposable endoscopes are typically made.

Furthermore, it is desirable to provide the front window of the tip part with shading members through two-stage two-component injection moulding of a transparent window material and an opaque shading material. This is to minimize glare from the built-in light sources to the camera of the insertion endoscope. One such tip part and manufacturing method is known from commonly-owned U.S. Patent No. 11,291,352.

While the tip housing in accordance with U.S. Patent No. 11,291,352 has shown to generally fulfil the above requirements, the joining of the two materials is not always optimal, *inter alia* because the interface between the transparent part, as seen from the distal end surface of the tip part, is formed only within the essentially cylindrical side wall of the housing away from the end wall, so that the area of the interface where the two materials bond together corresponds to the relatively thin housing side wall. This may have the effect that the electrical insulation becomes substandard, in turn leading to rejects during subsequent testing of the endoscopes, in turn, causing unnecessary costs. It furthermore, provides only a limited area for the two materials to fuse together and provide the necessary mechanical strength between the materials.

### SUMMARY

Based on this prior art it is an object of the invention to provide an endoscope, as set out in claim 1, with an improved housing for the tip part which does not suffer from the above drawbacks, and a manufacturing method of said endoscope according to claim 8.

According to a first aspect of the disclosure, this object is achieved by an endoscope with a distal tip part comprising a tip housing integrally moulded from a first material and a second material that is transparent. The tip housing has an end wall formed from the first material and the second material, and in the end wall the second material at least in part overlaps the first material. The tip housing can be moulded in a two step injection moulding process that yields a one-piece integrally moulded part.

In some embodiments, said housing comprises the end wall and a surrounding side wall with an internal end wall surface and an internal side wall surface so as to define at least one inner compartment accommodating an electronic vision device and at least one light source, where the end wall comprises an external end surface facing the exterior of the housing and the surrounding side wall comprises an external surrounding side surface facing the exterior of the housing, where the second material is provided as a part of the end wall so as to provide a vision window in the end wall covering the electronic vision device and a light emission window adapted to receive and transmit light from the at least one light source through the end wall, wherein, in the end wall, said second material overlaps at least partially said first material when viewed from the exterior towards the external end surface.

By creating such an overlap, the area over which the first and second materials contact each other and fuse together is increased, thus better ensuring proper fusion of the two materials. Furthermore, the distance along the fusion seam from the inside to the outside is increased, in turn, leading to a longer electrical paths, should defects in the fusion exist, hence further decreasing the risk of electrical breakdown.

According to a second aspect of the disclosure the object is achieved by a method in manufacturing an endoscope according to any one of the preceding claims wherein the tip is injection moulded in a two-stage injection moulding process in which, in the first stage, the first material is injected into a mould through a first single gate and in which, in the second stage said second material overlap is injected through a second single gate. Such method allows a cost-efficient way of manufacturing in particular the tip part of the endoscope.

According to a third aspect of the disclosure, the object is achieved by a system comprising a display device and an endoscope according to the first aspect of the disclosure connectable to the display device. In such a system the use of an endoscope according to the first aspect reduces the risk of electrical breakdown induced via the electrical connection between the endoscope and display device.

According to an embodiment of the first aspect of the disclosure, the second material is provided as one contiguous part so as to provide both said vision window and said light emission window. This allows the injection of the transparent material through a single gate only, during the injection moulding process.

According to an embodiment according to the first aspect of the disclosure, the second material is provided as one contiguous part providing part of the side wall and part of the end wall. This allows the moulding gate of the mould to be placed at a location away from front window so as to minimize any disturbance of the optical properties that may be caused by the moulding process.

According to an embodiment according to the first aspect of the disclosure, the overlap is formed as a step between the first and second materials. Using a step further increase the distance from the inside to the outside along the fusion seam and may provide large interface areas where the first and second material fuse together.

According to another embodiment according to the first aspect of the disclosure, the step comprises a first interface extending below and in parallel with the end surface. Having the interface extending below the end surface, increases the mechanical resistance of the fusion seam as the main forces acting on the end surface during insertion of the endoscope will be perpendicular to the interface.

According to a further embodiment of the first aspect of the disclosure, the step comprises a second interface extending essentially at a right angle to said first interface. This increased the area of the overall interface between the first and second material and further strengthens the fusion seam against shear forces.

According to a further embodiment of the first aspect of the disclosure, said overlap is arranged at a central part of the end surface away from the surrounding side wall when viewed from said end surface. This allows the enlarged interface are to be located in a place where it does not disturb other features, and hence allows for a relatively large and strong interface between the first and second materials.

According to a further embodiment according to the first aspect of the disclosure, the second material overlaps a moulding artefact in the first material. Thereby, moulding artefacts, such as defects and remnants from the gate of the first molding stage may be covered, in turn, providing the end wall with a smooth surface on which nothing may get caught and which is better at repelling residue that may block the vision or illumination.

According to yet another embodiment according to the first aspect of the disclosure, the tip is injection moulded in a two-stage injection moulding process in which, in the first stage, the first material is injected into a mould through a first single gate and in which, in the second stage said second material overlap is injected through a second single gate.

According to yet a further embodiment according to the first aspect of the disclosure, the second gate is arranged in conjunction with the side surface of the tip housing. That is to say at a location on the side surface, so that any residue from broken off runners at the gate will end be present on the side surface. This allows proper flow of the second material from the second gate into the front window part and the light guides which are preferably arranged mirror-symmetrical on either side of the central vision window, ensuring good filling and hence good optical properties, inter alia by keeping the single gate away from the front window surfaces.

According to still another preferred embodiment, the first gate is arranged in conjunction with the interface. That is to say at a location in the interface, so that any residue from broken off runners at the gate will end be present in the interface rather than on the outer surface of the housing. This allows central filling and good flow from a single gate that may later be covered by the second material injected elsewhere.

According to a fourth aspect of the disclosure, a method of making an endoscope is provided, the method comprising: injection moulding a tip housing comprising, after said injection moulding, an end wall and a surrounding side wall so as to define at least one inner compartment configured to accommodate an electronic vision device and at least one light source, the end wall comprising an end surface and the surrounding side wall comprising a side surface, the end wall and the side wall integrally moulded from a first material and a second material, the second material being a transparent material, wherein said injection moulding comprises, in a first stage, injecting the first material into a mould through a first single gate and, in a second stage, injecting the second material through a second single gate, and wherein, in the end wall, the second material overlaps at least partially the first material when viewed from the end surface. The present invention is set out in the appended claims.

### BRIEF DESCRIPTION OF THE DRAWINGS

The disclosure will now be made in greater detail based on non-limiting exemplary embodiments and with reference to the drawings on which:
Fig. 1 shows a system comprising a display device and an endoscope according to the disclosure connected thereto,
Figs. 1a and 1b show variations of the display device of Fig. 1,
Fig. 2 shows a tip housing used in the endoscope according to the disclosure,
Fig. 3 shows an exploded view of the tip housing of Fig. 2,
Fig. 4 shows a distal end view of the tip housing of Fig. 2,
Fig. 5 shows the inside of the tip housing of Fig. 2 as seen from the proximal end,
Fig. 6 shows a perspective front view of the first housing part,
Fig. 7 schematically shows the first housing part in a longitudinal section of a first configuration of an exemplary mould, and
Fig. 8 schematically shows the complete housing part in a longitudinal section of a second configuration of the exemplary mould.

### DETAILED DESCRIPTION

The present disclosure may be further understood with reference to the following description and appended drawings, wherein like elements are referred to with the same reference numerals.

Unless otherwise defined, all technical and scientific terms used herein have the same meaning as commonly understood by one of ordinary skill in the art. In case of conflict, the present document, including definitions, will control. Preferred methods and materials are illustrated below, although apparatuses, methods, and materials similar or equivalent to those illustrated herein may be used in practice or testing. All publications, patent applications, patents and other references mentioned herein are incorporated by reference in their entirety. The materials, methods, and examples disclosed herein are illustrative only and not intended to be limiting.

The terms "comprise(s)," "include(s)," "having," "has," "can," "contain(s)," and variants thereof, as used herein, are intended to be open-ended transitional terms that do not preclude the possibility of additional acts or structures. By contrast, the term "consists," as used herein, is intended to be a closed-ended transitional term that precludes the possibility of additional acts or structures.

The term "distal," as used herein, refers to a direction or position that is generally towards a target site, and the term "proximal," as used herein, refers to a direction or position that is generally away from the target site.

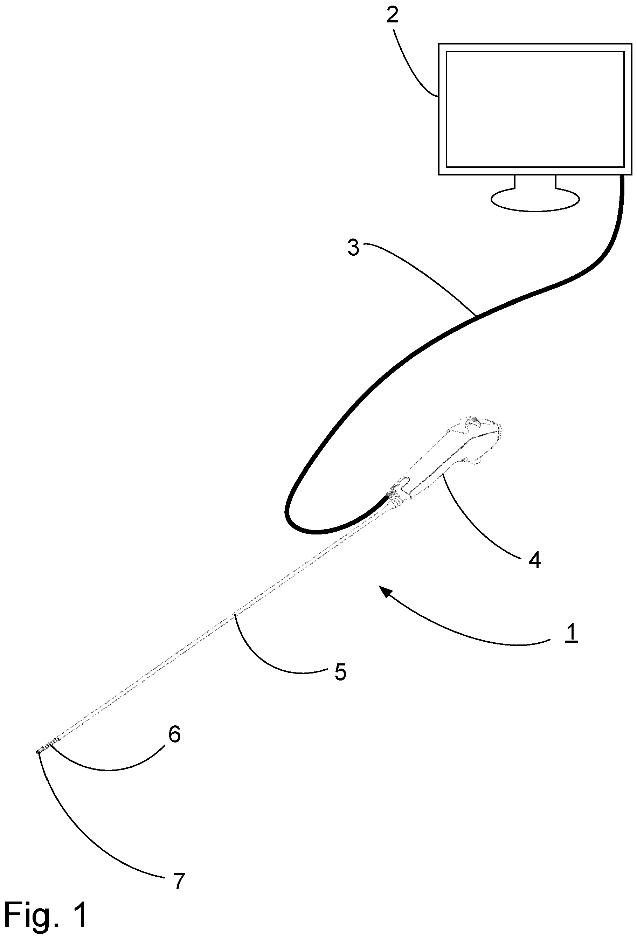

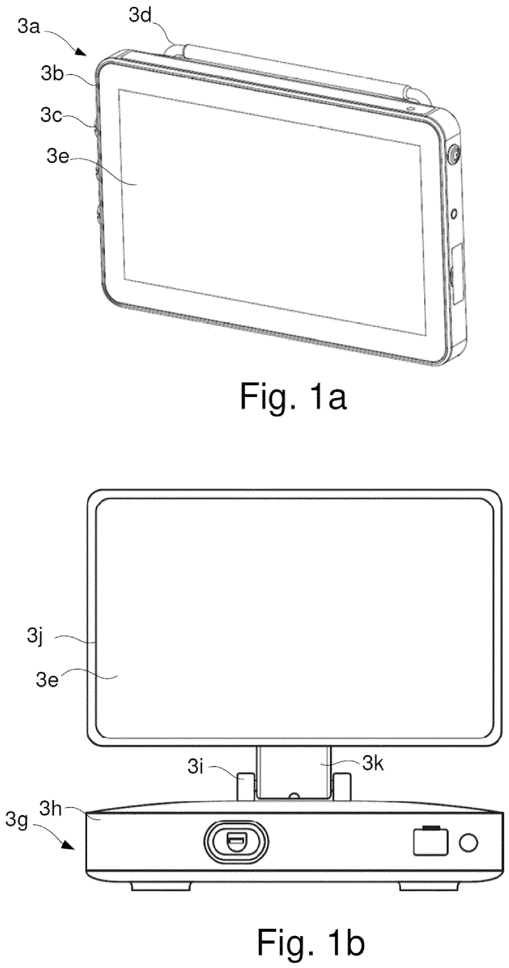

Turning first to Fig. 1, a visualization system 1 comprising an endoscope 2 according to the disclosure and a display device or video processing apparatus (VPA) 3 is shown. The endoscope 2 is connected to the display device 3 via a communications connection, which in the illustrated example is a cable 4. The cable 4 may also include power supply for the electronics in the endoscope 2 as well as channels for irrigation and suction etc. thus constituting what is commonly referred to as an umbilical.

The endoscope 2 in the illustrated example comprises a proximal handle 5 adapted to be gripped by a hand of an operator. From the handle 5 an insertion cord 6 extends towards the distal end of the endoscope 2. The insertion cord 6 comprises an insertion tube 6a and a bending section 6b extending therefrom. At the distal end of the endoscope 2 the insertion cord 6 includes a tip housing 7, typically constituting the most distal end of the endoscope 2. The focus of the present disclosure is on the tip housing 7, as illustrated in the subsequent figures, and the skilled person will understand that the remainder of the endoscope 2 may have a different design without deviating from the essence of the disclosure.

In one variation, shown in Fig. 1a, a VPA 3a includes a housing 3b, a receptacle 3c to receive a connector of the cable 4, and a handle 3d that also functions as a table stand when the monitor is positioned on a table or other support structure. The housing encloses in part a display screen 3e and circuitry operable to configure the live video captured by the image sensor of the camera module of the endoscope 2 to the requirements of the display screeen. The circuitry also generates a graphical user interface configured to enable the operator to control the image or video capture and presentation functions, as is known in the art.

In another variation, shown in Fig. 1b, a VPA 3g includes a housing 3h and a receptacle (not shown) to receive the connector of the cable 4 but does not include a display screen. The housing, in this variation, includes an optional display support interface 3i, which supports a display device 3j, having the display screen 3e, via a support arm 3k. The display device can be separated or removed from the monitor.

Both variations of the VPA may perform the same video processing functions. The processed video can be presented with a separate display device communicatively connected, via a wired or wireless connection, with the VPA 3, 3a, 3g. This enables placement of the display screen in a location distinct from the location of the VPA 3, 3a, 3g. This also enables use of a display device available in the operating room for other purposes.

A position interface functions to control the position of the insertion cord 6. The handle 4 is an example of a position interface and, unless stated otherwise, the terms are used interchangeably. The handle also functions to provide manual control actuators, e.g. knobs, levers, buttons, and the like, to steer the housing 7 and control instruments guided through the insertion cord. Alternatively, a different position interface can be provided that is connected to the insertion cord and is detachably connected to a robotic arm. The insertion cord thus extends from the robotic arm, and the endoscope is thus detachable from the robotic arm. The housing 7 is the same regardless of the position interface used. The robotic arm responds to signals, such as voice commands from the operator, to rotate, translate, and otherwise position the proximal end of the insertion cord, as an operator would do manually. The position interface can include control actuators, including manual control actuators. Alternatively or additionally, control actuators can be provided in or on the robotic arm or by the robotic system including the robotic arm, thereby potentially reducing the cost of the endoscope. Example control actuators include single axis actuators, including linear motion actuators. A linear motion actuator may comprise a threaded rod coupled to a threaded nut portion, in which a motor rotates the rod to translate the nut portion.

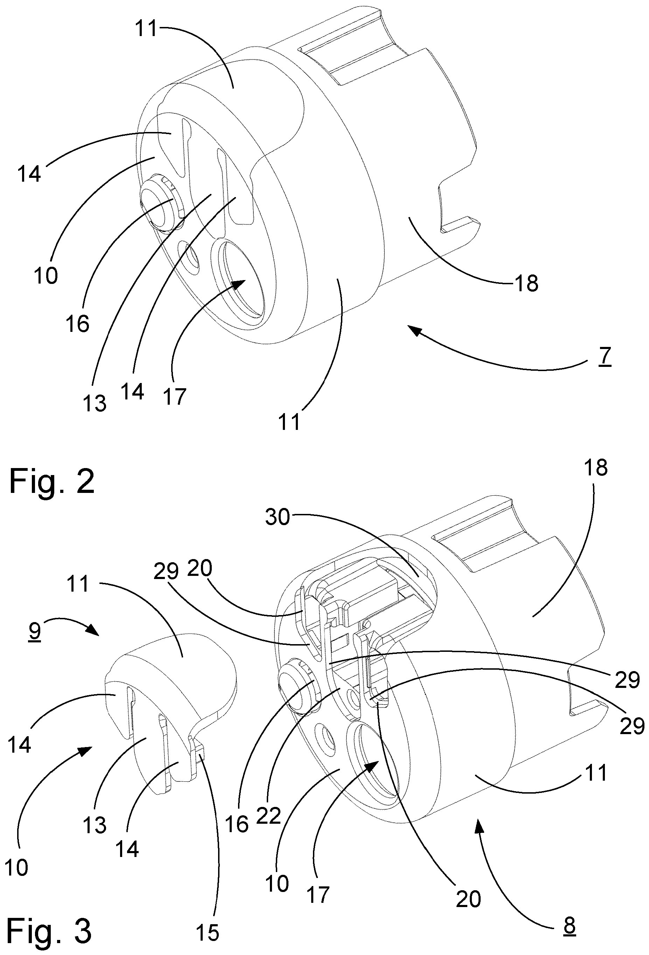

In Fig. 2 the tip housing 7 is shown in greater detail. The tip housing 7 is integrally moulded from a first material and a second material, so as to comprise a first housing part 8 and a second housing part 9. The second material forming the second housing part 9 is a transparent material. The first material forming the first housing part on the other hand is coloured or preferably opaque. The first and second materials are preferably polymer materials and suitable for injection moulding. The polymer of the first and second materials may be the same, i.e. the materials differing only in the filler, such as carbon black, coloured dye or lack of same.

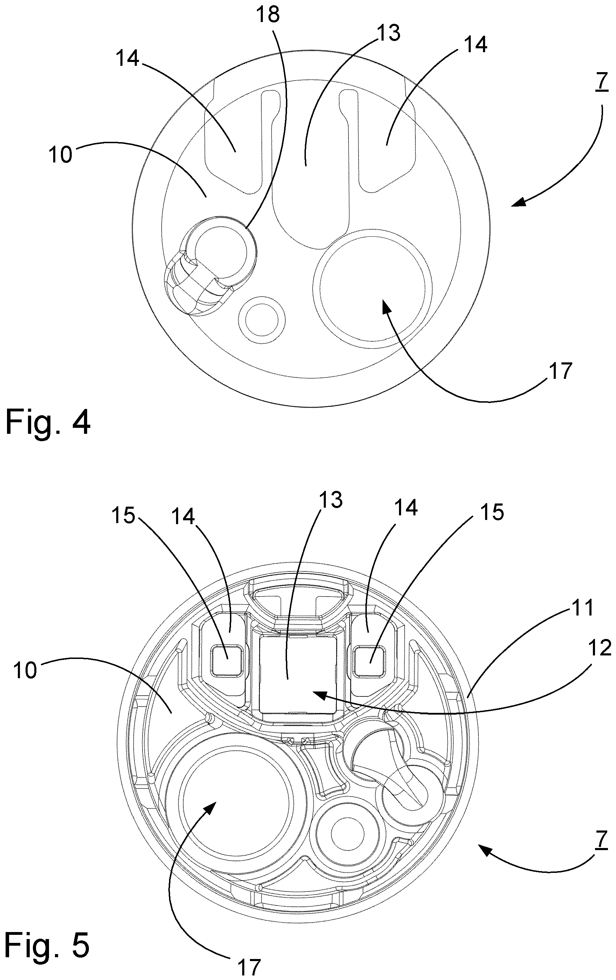

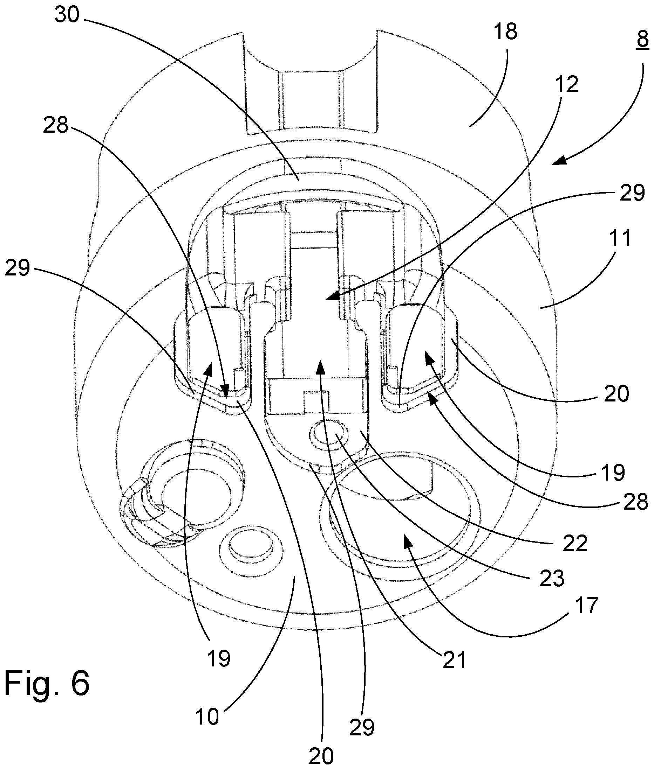

The housing 7 comprises an end wall 10 and a surrounding side wall 11 so as to define at least one inner compartment 12, best seen in Figs. 5 and 6. The inner compartment accommodates an electronic vision device and at least one light source, not shown. The end wall 10 comprises an external end surface and the surrounding side wall 11 comprises an external surrounding side surface, forming the exterior surface of the housing 7.

As can best be seen from Fig. 3, the second material is provided as one contiguous part providing part of the side wall 11 and part of the end wall 10 so as to provide a vision window 13 in the end wall traversing the optical axis of the electronic device (not shown) and at least one light emission window 14 positioned distally of the at least one light source. In the illustrated example there are two light emission windows 14 arranged mirror-symmetrically on either side of the vision window 13. The light emission windows 14, may each comprise a light guide 15 adapted to receive and transmit light from the at least one light source (not shown) through the end wall 10. The light guides 15 are adapted to shape the light emitted from the light sources in order to provide a desired light distribution withing the field of view of the endoscope 2, e.g. as described in EP3539451, incorporated herein by reference.

The end wall 10 may comprise additional features provided with the first housing part 8 such as a spray nozzle 16 and a working channel exit port 17 where the spray nozzle is adapted to spray water onto the vision window 13 and/or the light emission windows 14.

Towards the proximal end of the housing 7 the first housing part 8 may have a slightly recessed portion 18 to allow the bending section 6 to overlap the housing 7 and form a good connection when assembling the endoscope 2. On top of both the housing 7 and the bending section 6 an outer sleeve or covering is normally fitted, in order to avoid undesired fluid ingress into the bending section 6, which is normally a relatively open articulated structure.

Turning now to Fig. 6 details of the first housing part 8 as it would appear after a first moulding stage, i.e. before the second material is added in a second moulding stage, can better be seen. Since the first material is preferably opaque, apertures 19 allowing the light from the light sources within the housing 7 are provided in the end wall 10. These apertures 19 will be covered by the light emission windows 14 when in the second moulding stage. The apertures 19 have a cross-sectional area and a circumference allowing for the accommodation of the light guides 15, if provided, e.g. as an integral part of the light emission window 14. As can be seen the end wall comprises a transition section 28 through the end wall so that the light emission window area at the end surface is larger than the cross-sectional area of the aperture deeper within the end wall 10. This could be provided by flaring the walls of the aperture 19 out, but preferably this is done in a stepwise manner as illustrated. This provides plane surfaces 20 forming a first interface between the first and second materials within the end wall 10 of the final housing 7 after the second moulding step, so as to provide an increased adhesion or fusion area between the first material and the second material. The plane surfaces 20 are preferably arranged so that they are parallel with the final end surface 10 of the finished housing 7, thus also providing good manual support for the light emission windows 14. In other words the windows formed from the second material are provided with an undercut supported by the first material. Furthermore, the distance from the end surface outside the end wall 10 to the interior of the housing along the fusion seam through the wall, becomes greater than if the fusion seam was straight through the end surface perpendicular to the surface. In other words, the first interface between the first material and the second material becomes larger because of the overlap. Should defects exist the path for ingress of fluids and electrical breakdown along the fusion seam will be longer, and the risk of fluid penetration through the end wall and the risk of electrical breakdown accordingly reduced.

Similarly, i.e. for the same reasons, the passage 21 for light into the vision device may also be widened towards the end surface 10, preferably also in a stepwise manner providing an essentially plane surface 22 adjacent the through passage 21. The plane surface 22 is preferably also arranged to be parallel to the final end surface 10 of the finished housing 7. Furthermore, the plane surface 22 may be at least partially surrounded by a wall 29 perpendicular to the plane surface 22, hence forming the riser of the step. This surrounding wall 29 forms a second interface further increases the area and thus the mechanical strength of the overall interface between the first and second materials, in particular against shear forces.

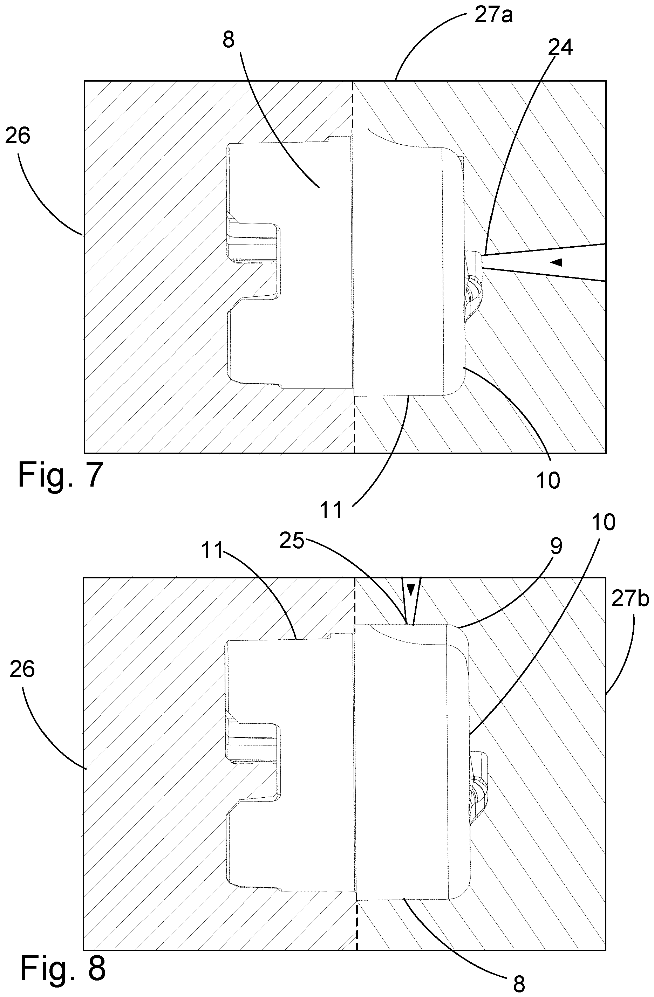

As can be seen the essentially plane surface 22 may comprise a defect in the form of remnants 23 of the gate 24 (cf. Fig. 7) for the polymer material into the mould cavity in the first moulding step. This allows the first housing part 8 to be moulded using only a single gate 24, the remnants of which may then be covered to provide a smooth end surface.

The contiguous transparent material may also form part of the side wall, overlapping in a similar stepwise manner a preferably curved surface 30 forming a further interface between the first and second materials.

As will be further explained below and seen from Fig. 8 the transparent second housing part 9 may also be moulded using a single gate 25 only. This gate 25 may be provided in the side wall 11 where defects from the gate 25 will not have any influence on the optical transmission properties of the light emission windows 14 and the vision window 13. Also, here a step may be provided in the wall 11 so as to provide a concentric surface 25 to support the second material and to increase the length of the fusion seam.

Turning now to Fig. 7, a longitudinal section through a first mould configuration for the first moulding stage is shown with the moulded first housing part 8 (not in section) placed in the mould cavity for illustration. Similarly, in Fig. 8 a longitudinal section of a second mould configuration for the second moulding stage is shown with the finished moulded housing 7 (not in section) placed in the mould cavity for illustration.

In Fig. 7 a first mould configuration comprising two separable halves 26, 27a forming a fist mould cavity corresponding to the first housing part 8, is shown. The first mould half 26 has a central convex shape that protruded into the cavity of the second half 27a so as to provide the interior volume of the first housing part 8 when moulded in the first stage of a two-stage two component injection moulding process. The first material is preferably injected through a single gate arranged close to a centre axis of the first housing part 8. Fig. 7 is schematical and details of sprues and runners leading to the gates or inlets of the mould cavity are not shown. When the first material has set the two halves are separated, preferably with the already set first material preferably remaining stuck in the first half 26. A new second mould configuration shown in Fig. 8 may then be provided by substituting the second mould part 27a with new second half 27b corresponding to the desired final shape of the housing 7. Remaining stuck in the first half 26, the already set first part thus fills the reconfigured moulding cavity partially, leaving only room for the second material to provide the transparent second housing part 9 with the transparent windows 13, 14 of the final housing 7. The second material may then be injected via a single gate so as to form a single contiguous part. This may be done with increased pressure so that the first material properly contacts the inner wall of the second mould part 27a and provides windows 13, 14 with no undesired defects. When the second material set the finished housing 7 may be removed from the mould.

Because the two single gates 24 and 25 are in different locations the second material may smoothly cover any remnants of the first gate of the mould part 27a in the finished housing 7.

## Claims

1. An endoscope with a distal tip part comprising a tip housing integrally moulded from a first material and a second material, the second material being a transparent material,
said housing comprising an end wall and a surrounding side wall with an internal end wall surface and an internal side wall surface so as to define at least one inner compartment accommodating an electronic vision device and at least one light source,
where the end wall comprises an external end surface facing the exterior of the housing and the surrounding side wall comprises an external surrounding side surface facing the exterior of the housing,
where the second material is provided as a part of the end wall so as to provide a vision window in the end wall covering the electronic vision device and a light emission window adapted to receive and transmit light from the at least one light source through the end wall,
wherein, in the end wall, said second material overlaps at least partially said first material when viewed from the exterior towards the external end surface
wherein the second material is provided as one contiguous part so as to provide both said vision window and said light emission window, and
wherein the second material is provided as one contiguous part providing part of the side wall and part of the end wall.

2. An endoscope according to claim 1, wherein the overlap is formed as a step between the first and second materials.

3. An endoscope according to any one of claims 1 or 2, wherein the step comprises an interface extending below and in parallel with the end surface.

4. An endoscope according to claim 3, wherein the step comprises a second interface extending essentially at a right angle to said first interface.

5. An endoscope according to any one of claims 1 to 4, wherein said overlap is arranged at a central part of the end surface away from the surrounding side wall when viewed from said end surface.

6. An endoscope according to any one of the preceding claims, wherein the light emission window comprises a light guide.

7. An endoscope according to anyone of the preceding claims wherein said second material overlaps a moulding artefact in the first material.

8. A method in manufacturing an endoscope according to any one of the preceding claims wherein the tip is injection moulded in a two-stage injection moulding process in which, in the first stage, the first material is injected into a mould through a first single gate and in which, in the second stage said second material overlap is injected through a second single gate.

9. A method according to claim 6, wherein said second material overlaps the location of said first gate.

10. A method according to any one of claims 8 or 9, wherein the second gate is arranged in conjunction with the side surface of the tip housing.

11. A system comprising a display device and an endoscope according to any one of claims 1 to 7 connectable to the display device.

## Patentansprüche

1. Endoskop mit einem distalen Spitzenteil, das ein Spitzengehäuse umfasst, das einstückig aus einem ersten Material und einem zweiten Material geformt ist, wobei das zweite Material ein transparentes Material ist,
wobei das Gehäuse eine Endwand und eine umgebende Seitenwand mit einer inneren Endwandfläche und einer inneren Seitenwandfläche umfasst, um mindestens ein Innenfach zu definieren, das eine elektronische Sichtvorrichtung und mindestens eine Lichtquelle aufnimmt,
wobei die Endwand eine äußere Endfläche umfasst, die dem Äußeren des Gehäuses zugewandt ist, und die umgebende Seitenwand eine äußere umgebende Seitenfläche umfasst, die dem Äußeren des Gehäuses zugewandt ist,
wobei das zweite Material als ein Teil der Endwand bereitgestellt ist, um ein Sichtfenster in der Endwand, das die elektronische Sichtvorrichtung bedeckt, und ein Lichtemissionsfenster bereitzustellen, das dazu ausgelegt ist, Licht von der mindestens einen Lichtquelle durch die Endwand zu empfangen und zu übertragen,
wobei das zweite Material in der Endwand das erste Material zumindest teilweise überlappt, wenn es von außen zu der äußeren Endfläche hin betrachtet wird,
wobei das zweite Material als ein zusammenhängendes Teil bereitgestellt ist, um sowohl das Sichtfenster als auch das Lichtemissionsfenster bereitzustellen, und
wobei das zweite Material als ein zusammenhängendes Teil bereitgestellt ist, das einen Teil der Seitenwand und einen Teil der Endwand bereitstellt.

2. Endoskop nach Anspruch 1, wobei die Überlappung als eine Stufe zwischen dem ersten und dem zweiten Material gebildet ist.

3. Endoskop nach einem der Ansprüche 1 oder 2, wobei die Stufe eine Grenzfläche umfasst, die sich unterhalb und parallel zu der Endfläche erstreckt.

4. Endoskop nach Anspruch 3, wobei der Schritt eine zweite Grenzfläche umfasst, die sich im Wesentlichen in einem rechten Winkel zu der ersten Grenzfläche erstreckt.

5. Endoskop nach einem der Ansprüche 1 bis 4, wobei die Überlappung an einem zentralen Teil der Endfläche von der umgebenden Seitenwand weg angeordnet ist, wenn sie von der Endfläche aus betrachtet wird.

6. Endoskop nach einem der vorhergehenden Ansprüche, wobei das Lichtemissionsfenster einen Lichtleiter umfasst.

7. Endoskop nach einem der vorhergehenden Ansprüche, wobei das zweite Material ein Formungsartefakt in dem ersten Material überlappt.

8. Verfahren zur Herstellung eines Endoskops nach einem der vorhergehenden Ansprüche, wobei die Spitze in einem zweistufigen Spritzgussverfahren spritzgegossen wird, bei dem in der ersten Stufe das erste Material durch ein erstes einzelnes Tor in eine Form injiziert wird und bei dem in der zweiten Stufe die zweite Materialüberlappung durch ein zweites einzelnes Tor injiziert wird.

9. Verfahren nach Anspruch 6, wobei das zweite Material die Position des ersten Tors überlappt.

10. Verfahren nach einem der Ansprüche 8 oder 9, wobei das zweite Tor in Verbindung mit der Seitenfläche des Spitzengehäuses angeordnet ist.

11. System, das eine Anzeigeeinheit und ein Endoskop nach einem der Ansprüche 1 bis 7 umfasst.

## Revendications

1. Endoscope avec une partie bout distale comprenant un boîtier de bout moulé de façon monobloc à partir d'un premier matériau et d'un second matériau, le second matériau étant un matériau transparent,
ledit boîtier comprenant une paroi d'extrémité et une paroi latérale périphérique avec une surface de paroi d'extrémité interne et une surface de paroi latérale interne afin de définir au moins un compartiment intérieur logeant un dispositif de vision électronique et au moins une source de lumière,
où la paroi d'extrémité comprend une surface d'extrémité externe faisant face à l'extérieur du boîtier et la paroi latérale périphérique comprend une surface latérale périphérique externe faisant face à l'extérieur du boîtier,
où le second matériau est prévu en tant que partie de la paroi d'extrémité afin de fournir une fenêtre de vision dans la paroi d'extrémité couvrant le dispositif de vision électronique et une fenêtre d'émission de lumière adaptée pour recevoir et transmettre de la lumière, provenant de l'au moins une source de lumière, à travers la paroi d'extrémité,
dans lequel, dans la paroi d'extrémité, ledit second matériau chevauche au moins partiellement ledit premier matériau en vue depuis l'extérieur vers la surface d'extrémité externe,
dans lequel le second matériau est prévu sous forme de partie contiguë afin de fournir à la fois ladite fenêtre de vision et ladite fenêtre d'émission de lumière, et
dans lequel le second matériau est fourni sous forme de partie contiguë fournissant une partie de la paroi latérale et une partie de la paroi d'extrémité.

2. Endoscope selon la revendication 1, dans lequel le chevauchement est sous forme d'épaulement entre les premier et second matériaux.

3. Endoscope selon l'une quelconque des revendications 1 ou 2, dans lequel l'épaulement comprend une interface s'étendant en dessous et en parallèle avec la surface d'extrémité.

4. Endoscope selon la revendication 3, dans lequel l'épaulement comprend une seconde interface s'étendant essentiellement à angle droit par rapport à ladite première interface.

5. Endoscope selon l'une quelconque des revendications 1 à 4, dans lequel ledit chevauchement est agencé dans une partie centrale de la surface d'extrémité éloignée de la paroi latérale périphérique en vue depuis ladite surface d'extrémité.

6. Endoscope selon l'une quelconque des revendications précédentes, dans lequel la fenêtre d'émission de lumière comprend un conduit de lumière.

7. Endoscope selon l'une quelconque des revendications précédentes, dans lequel ledit second matériau chevauche un produit de moulage dans le premier matériau.

8. Procédé dans la fabrication d'un endoscope selon l'une quelconque des revendications précédentes, dans lequel le bout est moulé par injection dans un processus de moulage par injection à deux étape dans lequel, dans la première étape, le premier matériau est injecté dans un moule à travers une première entrée unique et dans lequel, dans la seconde étape, le chevauchement dudit second matériau est injecté à travers une seconde entrée unique.

9. Procédé selon la revendication 6, dans lequel ledit second matériau chevauche l'emplacement de ladite première entrée.

10. Procédé selon l'une quelconque des revendications 8 ou 9, dans lequel la seconde entrée est agencée conjointement avec la surface latérale du boîtier de bout.

11. Système, comprenant un dispositif d'affichage et un endoscope selon l'une quelconque des revendications 1 à 7 connectable au dispositif d'affichage.
